(19) Europäisches Patentamt
European Patent Office
Office européen des brevets

(11) EP 1 092 415 A2

(12) **EUROPÄISCHE PATENTANMELDUNG**

(43) Veröffentlichungstag:
**18.04.2001 Patentblatt 2001/16**

(51) Int. Cl.7: **A61K 7/00**, A61K 7/42, A61K 7/48

(21) Anmeldenummer: **00121318.0**

(22) Anmeldetag: **09.10.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **15.10.1999 DE 19949826**

(71) Anmelder:
**Beiersdorf Aktiengesellschaft**
**20245 Hamburg (DE)**

(72) Erfinder:
- **Dörschner, Albrecht**
  **20146 Hamburg (DE)**
- **Knüppel, Anja**
  **20257 Hamburg (DE)**

(54) **Kosmetische und dermatologische Lichtschutzformulierungen in Form von O/W-Makroemulsionen oder O/W-Mikroemulsionen, mit einem Gehalt an Dihydroxyaceton**

(57) Sprühbare Öl-in-Wasser-Emulsionen, insbesondere O/W-Mikroemulsionen, enthaltend anorganische PIgmente Emulgatoren, deren Lipophilie entweder vom pH-Wert oder von der Temperatur abhängig ist, und eine wirksame Menge an Dihydroxyaceton.

Fig. 1

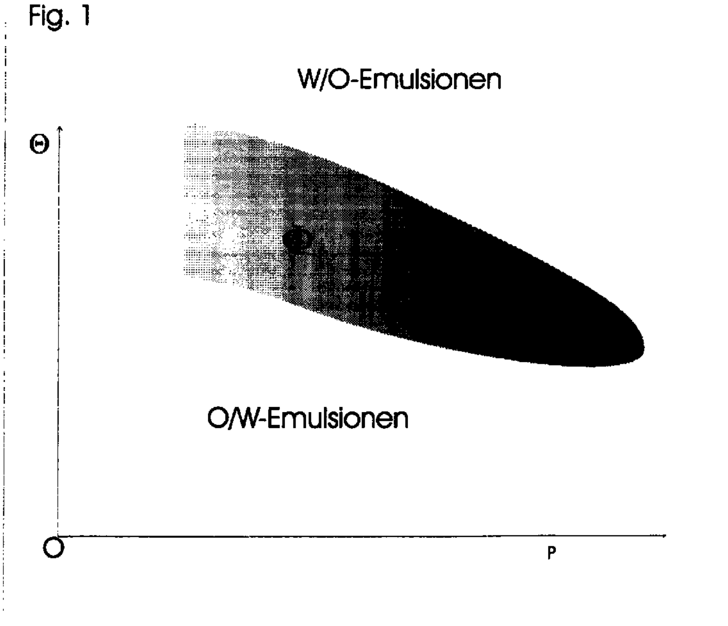


EP 1 092 415 A2

**Beschreibung**

Die vorliegende Erfindung betrifft kosmetische und dermatologische Zubereitungen zur Bräunung der Haut, insbesondere solche welche gleichzeitig Schutz gegen UV-Strahlung bieten.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

UV-A-Strahlung kann ferner Hautschädigungen hervorrufen, indem das hauteigene Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Die Pigmentierung der menschlichen Haut, wird im wesentlichen durch die Gegenwart von Melanin bewirkt. Melanin und dessen Abbauprodukte (Melanoide), Carotin, Durchblutungsgrad sowie die Beschaffenheit und Dicke des Stratum corneum und andere Hautschichten lassen Hautfarbtöne von praktisch weiß (bei verringerter Füllung oder bei Fehlen der Blutgefäße) oder gelblich über helibraun-rötlich, bläulich bis braun verschiedener Nuancen und schließlich beinahe schwarz erscheinen. Die einzelnen Hautregionen zeigen aufgrund unterschiedlicher Melanin-Mengen unterschiedliche Tiefe der Farbtönung.

Das natürliche Melanin schützt die Haut vor eindringender UV-Strahlung. Die Anzahl der in den Melanocyten produzierten Melanin-Granula entscheidet über Hell- od. Dunkelhäutigkeit. Bei starker Pigmentierung (z.B. bei Farbigen, aber auch bei Hellhäutigen nach einiger UV-Bestrahlung) ist Melanin auch im Stratum spinosum und sogar im Stratum corneum festzustellen. Es schwächt die UV-Strahlung um bis zu ca. 90%, bevor diese das Corium erreicht.

Je nach Lichtempfindlichkeit werden in der Regel folgende Hauttypen unterschieden:

| | |
|---|---|
| Hauttyp I | bräunt nie, bekommt immer einen Sonnenbrand. |
| Hauttyp II | bräunt kaum, bekommt leicht einen Sonnenbrand. |
| Hauttyp III | bräunt durchschnittlich gut. |
| Hauttyp IV | bräunt leicht und anhaltend, bekommt fast nie Sonnenbrand. |
| Hauttyp V | dunkle, oft fast schwarze Haut, bekommt nie Sonnenbrand. |

Die natürliche Abschirmung der schädlichen UV-Strahlung ist ein handfester Vorteil der natürlichen Hautbräunung. Seit einigen Jahrzehnten gilt darüberhinaus eine „gesunde" Hautfarbe als Zeichen von insbesondere sportlicher Aktivität und wird daher von einer breiten Verbraucherschicht als erstrebenswert erachtet. Vertreter der Hauttypen I und II, die sich einer solchen Hauttönung erfreuen wollen, sind daher ohnehin auf selbstbräunende Präparate angewiesen. Aber auch Vertreter des Hauttyps III, die sich nicht allzusehr den Risiken des Sonnenbades aussetzen und trotzdem gebräunt aussehen wollen, sind dankbare Zielgruppen für selbstbräunende Zubereitungen.

Künstliche Hautbräunung läßt sich auf kosmetischem bzw. medzinischem Wege bewirken, wobei im wesentlichen folgende Ansätze eine Rolle spielen:

Durch regelmäßige Einnahme von Carotin-Präparaten wird Carotin wird im Unterhaut-Fettgewebe gespeichert, die Haut färbt sich allmählich orange bis gelbbraun.

Mit Hilfe abwaschbarer Make-up-Präparate kann eine leichte Hauttönung erzielt werden (z.B. Extrakte aus frischen grünen Walnußschalen, Henna)

Die Anfärbung kann auch auf dem Wege der chemischen Veränderung der Hornschicht der Haut mit sogenannten selbstbräunenden Zubereitungen erfolgen. Wichtigster Wirkstoff ist das Dihydroxyaceton (DHA). Die auf diese Weise erzielte Hautbräunung ist nicht abwaschbar und wird erst mit der normalen Abschuppung der Haut (nach ca. 10-15 Tagen) entfernt.

Dihydroxyaceton zeichnet sich durch die Struktur

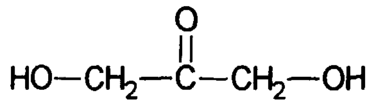

aus. Dihydroxyaceton (1,3-Dihydroxypropan-2-on) stellt einen farblosen Festkörper mit charakteristischem Geruch dar, In frisch bereiteten wäßrigen Lösungen liegt es als Dimeres vor, welches durch Erwärmen in die Monomeren zerfällt. Es kann als Ketotriose bezeichnet werden und reagiert als reduzierender Zucker mit den Aminosäuren der Haut bzw. den freien Amino- und Imino-Gruppen des Keratins über eine Reihe von Zwischenstufen im Sinne einer Maillard-Reaktion zu braungefärbten Stoffen, sogenanten Melanoiden, welche gelegentlich auch Melanoidine genannt werden.

Ein Nachteil der Bräunung mit Dihydroxyaceton liegt dann, daß die mit ihm gebräunte Haut im Gegensatze zu „sonnengebräunter" Haut nicht gegen Sonnenbrand geschützt ist.

Ein weiterer Nachteil von Dihydroxyaceton besteht dann, daß es, insbesondere unter dem Einfluß ultravioletter Strahlung, wenn auch in meist geringen Mengen Formaldehyd abspaltet. Es war daher ein dringender Bedarf Wege aufzuweisen, auf welchen der Zersetzung von Dihydroxyaceton wirksam begegnet werden kann.

Den Nachteilen des Standes der Technik abzuhelfen, war also Aufgabe der vorliegenden Erfindung.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigknstalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die Metastabilität von Emulsionen gewährleisten zu können, sind in der Regel grenzflächenaktive Substanzen, also Emulgatoren, nötig. An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen. So ist bekannt, daß bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wäßrigen Phase dispergierte Öltröpfchen, ähnlich einer O/W-Emulsion, aufweisen. Voraussetzung dafür kann sein, daß die kontinuierliche wäßrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes

der Technik an einer befriedigend großen Vielfalt solcher Emulgatoren, welche dann auch das Anwendungsspektrum entsprechend milder und hautverträglicher kosmetisctier Zubereitungen deutlich verbreitem würde.

So war eine weitere Aufgabe der vorliegenden Erfindung, kosmetische bzw. dermatologische Zubereitungen mit hervorragenden hautpflegenden Eigenschaften zur Verfügung zu stellen.

Natürlich ist dem Fachmann eine Vielzahl von Möglichkeiten bekannt, stabile O/W-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Ölewie die in handelsüblichen Produkten sonst häufig verwendeten Pflanzenöle - ausreichend stabilisiert sind, gibt es daher zur Zeit auf dem Markt nicht.

Unter dem Begriff „Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert heute diese sogenannte dynamische Viskosität nach $\eta$=t/D als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa • s).

Der Quotient $\nu=\eta/p$ aus der dynamischen Viskosität $\eta$ und der Dichte $\rho$ der Flüssigkeit wird als kinematische Viskosität $\nu$ bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens Newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen. In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta=\frac{2r^2(\rho_K-\rho_{Fl})\cdot g}{9\cdot v}$$

bestimmbar ist, wobei

r = Radius der Kugel, v = Fallgeschwindigkeit, $\rho_K$ = Dichte der Kugel, $\rho_{Fl}$ = Dichte der Flüssigkeit und g = Fallbeschleunigung.

O/W-Emulsionen mit einer geringen Viskosität, die eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Eine Aufgabe der vorliegenden Erfindung war es, Zubereitungen zur Verfügung zustellen, welche eine sehr geringe Viskosität haben und nicht die Nachteile des Standes der Technik aufweisen.

Die Definition für den HLB-Wert ist für Polyolfettsäureester gegeben durch die Formel I

$$HLB=20*(1-S/A)$$

Für eine Gruppe von Emulgatoren, deren hydrophiler Anteil nur aus Ethylenoxideinheiten besteht, gilt die Formel II

$$HLB = E/5$$

wobei

S = Verseifungszahl des Esters,

A = Säurezahl der zuruckgewonnen Säure

E = Massenanteil Ethylenoxid (in %) am Gesamtmolekül

bedeuten.

Emulgatoren mit HLB-Werten von 6-8 sind im allgemeinen W/O-Emulgatoren, solche mit HLB-Werten von 8-18 sind im allgemeinen O/W-Emulgatoren.

Literatur: "Kosmetik - Entwicklung, Herstellung und Anwendung kosmetischer Mittel"; W.Umbach (Hrsg.), Georg Thieme Verlag 1988.

Hydrophile Emulgatoren (mit hohen HLB-Werten) sind in der Regel O/W-Emulgatoren. Demgemäß sind hydrophobe oder lipophile Emulgatoren (mit niedrigen HLB-Werten) in der Regel W/O-Emulgatoren.

Die US-Patentschrift 4,931,210 beschreibt ein Verfahren zur Herstellung von W/O/W-Emulsion, wobei als Emulgatoren Polyglycerinpolyricinoleate verwendet werden.

Die Tröpfchendurchmesser der gewöhnlichen „einfachen", also nichtmultiplen Emulsionen liegen im Bereich von ca 1 µm bis ca. 50 µm. Solche „Makroemulsionen" sind, ohne weitere färbende Zusätze, milchigweißgefärbt und opak. Feinere „Makroemulsionen", deren Tröpfchendurchmesser im Bereich von ca. $10^{-1}$ µm bis ca. 1 µm liegen, sind, wiederum ohne färbende Zusätze, bläulichweißgefärbt und opak. Solche „Makroemulsionen" haben für gewöhnlich hohe Viskosität.

Mizellaren und molekularen Lösungen mit Partikeldurchmessern kleiner als ca. $10^{-2}$ µm, die allerdings nicht mehr als echte Emulsionen aufzufassen sind, ist vorbehalten, klar und transparent zu erscheinen.

Der Tröpfchendurchmesser von Mikroemulsionen dagegen liegt im Bereich von etwa $10^{-2}$ µm bis etwa $10^{-1}$ µm. Mikroemulsionen sind transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von „Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Es ist bekannt, daß hydrophile Emulgatoren bei steigender Temperatur ihr Löslichkeitsverhalten von wasserlöstich zu fettlöslich ändern. Der Temperaturbereich, in dem die Emulgatoren ihre Löslichkeit geändert haben, wird Phaseninversionstemperaturbereich (PIT) genannt.

T.J.Lin, H.Kurihara und H.Ohta (Journal of the Society of Cosmetic Chemists 26, S. 121 - 139, März 1975 zeigen bei unpolaren Ölen, daß im Bereich der PIT extrem instabile multiple Emulsionen vorliegen können.

Aufgabe der vorliegenden Erfindung war daher, diesen Übelständen Abhilfe zu schaffen.

Es hat sich überraschenderweise gezeigt, und darin liegt die Lösung der Aufgaben begründet, daß das Öl-in-Wasser-Emulsionen, insbesondere O/W-Mikroemulsionen

(a) enthaltend mindestens einen Emulgator (Emulgator A), gewählt aus der Gruppe der Emulgatoren mit folgenden Eigenschaften

- ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
- ihre Lipophilie ist abhängig von der Temperatur, dergestalt, daß die Lipophilie mit steigender Temperatur zunimmt und deren Hydrophilie mit sinkender Temperatur zunimmt,

(b) ferner gegebenenfalls weitere in der Ölphase oder der Wasserphase lösliche lösliche oder dispergierbare Substanzen, darunter bevorzugt solche, gewählt aus der Gruppe der nicht unter die Definition des Emulgators A fallende Emulgatoren, insbesondere solche, die vorwiegend als W/O-Emulgatoren wirken,
(c) eine wirksame Menge an Dihydroxyaceton,

den Nachteilen des Standes der Technik abhelfen.

Sofern die Phaseninversion im Sinne der vorliegenden Erfindung im wesentlichen durch Vanation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Es ist, wenn die Phaseninversion im wesentlichen durch Variation der Temperatur eingeleitet wird, durchaus vorteilhaft, auf weitere, nicht unter die Definition des Emulgators A fallende, weitere Emulgatoren, namentlich W/O-Emulgatoren, zu verzichten.

Die Gesamtmenge an Dihydroxyaceton in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Sofern die Phaseninversion im wesentlichen durch Variation des pH-Wertes eingeleitet wird, sind O/W-Emulsionen, insbesondere O/W-Mikroemulsionen, erhältlich. Es ist, wenn die Phaseninversion im wesentlichen durch Variation des pH-Wertes eingeleitet wird, durchaus vorteilhaft, einen oder mehrere weitere, nicht unter die Definition des Emulgators A fallende, weitere Emulgatoren, namentlich W/O-Emulgatoren, einzusetzen.

Erfindungsgemäß können O/W-Mikroemulsionen erhalten werden, wenn der Ölphasenanteil unter etwa 20 Gew.-%, insbesondere unter etwa 15 Gew.-%, bezogen auf das Gesamtgewicht der Zubereitung liegt, weniger als etwa 5 Gew.-% eines zusätzlichen, nicht unter die Definition des Emulgators A fallenden W/O-Emulgators vorliegen und/oder wenn die Ölphase einen hohen Anteil an polaren Ölen aufweist.

Erfindungsgemäß können O/W-Emulsionen („Makroemulsionen") erhalten werden, wenn weniger als etwa 5 Gew.-% eines zusätzlichen nicht unter die Definition des Emulgators A fallenden W/O-Emulgators und mehr als etwa 20 Gew.-% einer polaren Ölphase vorliegen.

Vorteilhaft können zusätzliche Gelbildner (z.B. Carbopole, Xanthangummi, Cellulosederivate) eingesetzt werden.

Es ist dabei im Einzelfalle möglich, daß die vorgenannten Konzentrationsgrenzen leicht über- oder unterschritten werden und dennoch die betreffenden Emulsionstypen erhalten werden, Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Es ist, wenn die Phaseninversion im wesentlichen durch Variation der Temperatur eingeleitet wird, durchaus vorteilhaft, wenngleich nicht unabdingbar, auf weitere, nicht unter die Definition des Emulgators A fallende, weitere Emulgatoren, namentlich W/O-Emulgatoren, zu verzichten.

Sofern die Phaseninversion im wesentlichen durch Variation des pH-Wertes eingeleitet wird, sind O/W-Emulsionen, insbesondere O/W-Mikroemulsionen, aber auch O/W/O-Emulsionen erhältlich. Es ist, wenn die Phaseninversion im wesentlichen durch Variation des pH-Wertes eingeleitet wird, durchaus vorteilhaft, einen oder mehrere weitere, nicht unter die Definition des Emulgators A fallende, weitere Emulgatoren, namentlich W/O-Emulgatoren, einzusetzen.

Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Es hat sich in erstaunlicher Weise herausgestellt, daß das oder die erfindungsgemäß eingesetzten Pigmentpartikel als Festkörper, und zwar gewissermaßen „verkapselt", nämlich abgetrennt von anderen Bestandteilen der Zubereitungen vorliegen, in welchen sie teilweise sogar begrenzte Löslichkeit besitzen können. Es wird angenommen, daß die Festkörperpartikel der schwerlöslichen UV-Filtersubstanzen durch das erfindungsgemäße Einarbeitungsverfahren einen Hüllfilm erhalten, welcher vermutlich als wesentlichen Bestandteil Emulgatormolekeln enthält.

Erfindungsgemäß kann die Rekristallisation des oder der erfindungsgemäß eingesetzten s-Triazinderivate verhindert werden. Darüberhinaus sind erfindungsgemäß Lichtschutzzubereitungen erhältlich, welche vorzügliche Anwendungseigenschaften aufweisen.

In Fig. 1 wird eine stark vereinfachte Darstellung eines Phasendiagrammes wiedergegeben. Der variable Parameter P wird gegen die Temperatur $\theta$ als zweite Variable aufgetragen. P stellt dabei einen Konzentrationsparameter dar, entweder den Anteil der Ölphase, den Anteil der Wasserphase oder die Konzentration eines Emulgators oder eines Emulgatorgemisches. Für erfindungsgemäße Systeme gilt dabei, daß bei niedrigeren Temperaturen eine O/W-Emulsion vorliegt und bei Erhöhung der Temperatur der Phaseninversionsbereich durchlaufen werden kann. Bei weiterer Erhöhung der Temperatur werden W/O-Emulsionen beobachtet. Die Struktur des Systems im Phaseniriversionsbereich ist dem Anschein nach nicht kritisch für die vorliegende Erfindung. Denkbar ist beispielsweise, daß im Phaseninversionsbereich lamellare Phasen, bikontinuierliche Phasen, kubische, hexagonale bzw. invers hexagonale Phasen vorliegen, auch daß der Phaseninversionsbereich aus mehreren gleichartigen oder mehr oder weniger unterschiedlichen Phasen zusammengesetzt ist.

Der Phaseninversionsbereich läßt sich mathematisch darstellen als Punktmenge innerhalb des geradlinigen Koordinatensystems $\Sigma$, welches durch die Größen Temperatur, der Konzentration eines geeigneten Emulgators bzw. eines Emulgatorengemisches in der Zubereitung sowie die jeweiligen Konzentrationen der Ölphase und der Wasserphase gebildet wird, gemäß:

$$\Sigma = \{O, \theta, m, H, W\},$$

mit

O - Koordinatenursprung
θ - Temperatur
m - Konzentration des Emulgators/Emulgatorengemisches
H - Konzentration der Ölphase
W - Konzentration der Wasserphase

Dabei muß genaugenommen natürlich in einem mehrkomponentigen Emulgatorensystem der Beitrag $m_i$ jedes einzelnen Emulgators zur Gesamtfunktion berücksichtigt werden, was bei einem i-komponentigen Emulgatorensystem zur Beziehung

$$\Sigma = \{ O, \theta, m_1, m_2, ..., m_i, H, W\}$$ führt.

Der Phaseninversionsbereich Φ stellt dabei im mathematischen Sinne ein zusammenhängendes Gebiet oder eine Vielzahl zusammenhängender Gebiete innerhalb des Koordinatensystems Σ dar. Φ repräsentiert die Gesamtmenge der Koordinatenpunkte $K(\theta, a, m_1, m_2, ..., m_i, H, W)$, welche erfindungsgemäße Gemische aus Wasserphase der Konzentration W, Ölphase der Konzentration H, i erfindungsgemäßen Emulgatoren der Konzentration $m_i$ bei der Temperatur θ bestimmen, und für welche gilt, daß beim Übergang von einer Koordinate $K_1 \notin \Phi$ zu einer Koordinate $K_2 \in \Phi$ Phaseninversion eintritt, wie in Fig. 2 beschrieben.

Unerheblich ist dabei, ob der Phaseninversionsbereich eines gegebenen Systems ein einziges zusammenhängendes (i + 3)-dimensionalen Gebiet darstellt oder aus mehreren zusammenhängenden, aber voneinander getrennten solchen Gebieten besteht, also mehreren Phaseninversionsbereichen eines gegebenen Systems entsprechend. Im Rahmen der hiermit vorgelegten Offenbarung wird daher stets verallgemeinernd von „dem" oder „einem" Phaseninversionsbereich gesprochen, auch bei Vorliegen zweier oder mehrerer voneinander getrennter solcher Bereiche.

Als variable Koordinaten in Fig.2 werden Temperatur θ und die der vorgeschilderte Konzentrationsparameter P angegeben, wobei offengelassen bleiben kann, um welchen speziellen Konzentrationsparameter es sich handelt. Beim Übergang von $K_1$ nach $K_2$ wird lediglich die Temperatur erhöht, die anderen Variablen werden konstant gehalten.

Unter den erfindungsgemäßen Bedingungen ist dieser Prozeß nicht reversibel, d.h., kehrt das System von der Koordinate $K_2 \in \Phi$ wieder zur Koordinate $K_1 \notin \Phi$ zurück, können erfindungsgemäße transparente O/W-Mikroemulsionen erhalten werden.

Die Praxis der Herstellung einer erfindungsgemäßen Mikroemulsion besteht demgemäß vorteilhaft darin, nach Auswahl geeigneter Rohstoffe, d.h., Wasser- und Ölphase, ein oder mehrere erfindungsgemäß verwendete O/W-Emulgatoren, letzterer oder letztere vorliegend in Konzentrationen, bei welchen Phaseninversion für das gegebene Gemisch möglich ist, und gegebenenfalls weitere Substanzen, die Einzelkomponenten unter Rühren zusammenzugeben, durch Erhöhung der Temperatur des Gemisches Phaseninversion herbeizuführen, hernach unter fortwährendem Rühren das Gemisch auf Raumtemperatur abkühlen zu lassen.

Es ist dabei aber auch möglich, mehrere Parameter zugleich zu variieren, wie in Fig.3 angezeigt. In Fig. 3 wird die Konzentration der Wasserphase gegen die Temperatur aufgetragen. Ausgehend von der Koordinate $K_1 \notin \Phi$ können durch Erhöhung der Temperatur, unter Beibehaltung aller anderen Parameter, die Koordinaten $K_2 \notin \Phi$ und $K_4 \notin \Phi$ erreicht werden bzw. $K_3 \in \Phi$. Ausgehend von den Koordinaten $K_3$ und $K_4$ können durch Senken der Temperatur, unter Beibehaltung aller anderen Parameter, zurück zur Koordinate $K_1$ erfindungsgemäße O/W-Mikroemulsionen erhalten werden.

Ausgehend von den Koordinaten $K_3$ und $K_4$ kann durch Senken der Temperatur, und durch zusätzliche Variation der Konzentration der Ölphase, in Fig.3 durch Zugabe von Wasser, die Koordinate $K_5$ erreicht und können erfindungsgemäße O/W-Mikroemulsionen erhalten werden.

Es ist angesichts der Fig. 3 konsequent, daß, ausgehend von der Koordinate $K_4$, obwohl dieser außerhalb des Phaseninversionsbereiches befindlich ist, Systeme erhalten werden können, ähnlich denen, die von $K_3$ ausgehen, da ja auch ausgehend von $K_4$ bei Senkung der Temperatur der Phaseninversionsbereich zwangsläufig durchschritten werden muß.

Auch ausgehend von der Koordinate $K_1$ kann durch Variation der Konzentration der Wasserphase, also beispielsweise durch Zugabe von Wasser, wie in Fig. 3 aufgeführt, die Koordinate $K_5$ erreicht und können erfindungsgemäße O/W-Mikroemulsionen erhalten werden. Dazu muß allerdings vorausgeschickt werden, daß in diesem Falle bereits eine erfindungsgemäße O/W-Mikroemulsion, gewissermaßen als Konzentrat, vorliegen muß, welches dann durch Verdünnen zu einer erfindungsgemäßen O/W-Mikroemulsion veränderter Zusammensetzung umgesetzt wird.

Es war jedoch nach allem erstaunlich und verfügt daher über eigenständige erfinderische Tätigkeit, daß auch ausgehend von der Koordinate $K_2$, welche außerhalb des Phaseninversionsbereiches liegt, sei es bei einfacher Variation der Temperatur zurück zur Koordinate $K_1$ oder zusätzlicher Variation der Konzentration der Ölphase, also beispielsweise durch zusätzliches Verdünnen mit einer Wasserphase zur Koordinate $K_5$, erfindungsgemäße O/W-Mikroemulsionen erhältlich sind, ohne daß Phaseninversion durchlaufen wird. Dies geschieht vorteilhaft auf die Weise, daß ein Gemisch aus den Grundkomponenten, umfassend Wasserphase, Ölphase, einen oder mehrere der erfindungsgemäß verwendeten O/W-Emulgatoren, gewünschtenfalls einen oder mehrere W/O-Emulgatoren, sowie gewünschtenfalls weitere Hufs-, Zusatz- und/oder Wirkstoffe, welche unterhalb des Phaseninversionstemperaturbereiches eine O/W-Emulsion bilden, auf eine Temperatur bringt,

- bei welcher die in der Ölphase löslichen Komponenten entweder gelöst oder zumindest in geschmolzenem Zustande vorliegen
- und welche mindestens der Schmelztemperatur der höchstschmelzenden, nicht in gelöstem Zustande vorliegenden Ölkomponente entspricht,
- welche unterhalb des Phaseninversionstemperaturbereiches des Systemes liegt,

und die entstandene O/W-Emulsion unter Bildung einer O/W-Mikroemulsion hernach auf Raumtemperatur abkühlt. Dies geschieht bevorzugt unter Rühren.

Dieses erfindungsgemäße Verfahren ist besonders gut geeignet, wenn den erfindungsgemäßen O/W-Mikroemulsionen wärmeempfindliche oder leichtflüchtige Substanzen einverleibt werden sollen. Darüberhinaus ist dieses bei relativ niedrigen Temperaturen durchzuführende Verfahren gegenüber üblichen Verfahren energiesparend.

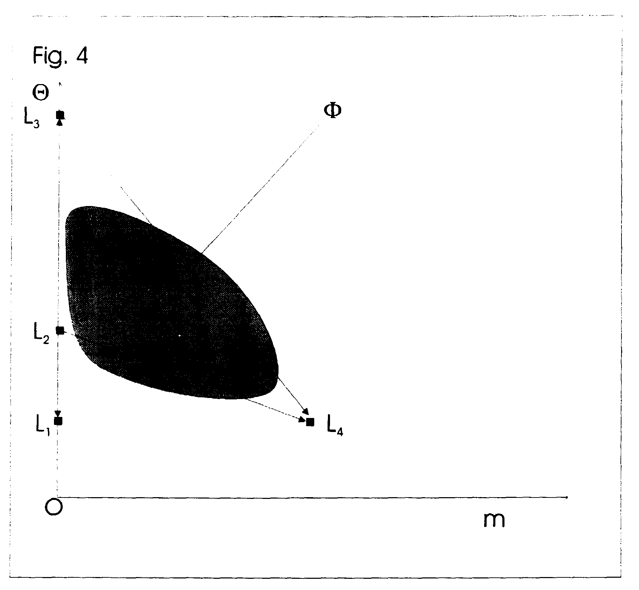

In Fig. 4 wird der Fall beschrieben, in welchem in der Koordinate $L_1$ zunächst kein erfindungsgemäßer O/W-Emulgator vorliegt, und in welchem das System durch Erhöhen der Temperatur auf eine Koordinaten $L_3 \notin \Phi$ oder auf eine Koordinate $L_2 \notin \Phi$ gebracht wird. Selbstverständlich kann die Koordinate $L_2$ auch durch Abkühlen eines in der Koordinate $L_3$ vorliegenden Systems erreicht werden. Die Koordinaten $L_2$ und $L_3$, in denen beispielsweise W/O-Emulsionen vorliegen können, unterscheiden sich prinzipiell lediglich dadurch, daß die $L_3$ zugeordnete Temperatur höher ist als jede Temperatur, die dem Phaseninversionstemperaturbereiche zugeordnet werden kann.

Die Gegenwart eines zusätzlichen W/O-Emulgators für Systeme, die in Fig. 4 symbolisiert werden, ist nicht unbedingt erforderlich, allerdings vorteilhaft. Zugabe eines erfindungsgemäßen O/W-Emulgators oder mehrerer solcher Emulgatoren in den Koordinaten $L_2$ oder $L_3$, bei Senkung der Temperatur, befördert das System zur Koordinate $L_4$, bei welcher dann eine erfindungsgemäße O/W-Mikroemulsion vorliegt.

Eine weitere vorteilhafte Ausführungsform des erfindungsgemäßen Verfahrens besteht demgemäß darin, nach der Auswahl geeigneter Rohstoffe, d.h., Wasser- und Ölphase und gegebenenfalls weitere Substanzen, die Einzelkomponenten unter Rühren auf eine Temperatur zu bringen, bei welcher Phaseninversion für das gegebene Gemisch möglich ist, und durch Zugabe des erfindungsgemäß verwendeten O/W-Emulgators oder der erfindungsgemäß verwendeten O/W-Emulgatoren zum Gemisch Phaseninversion herbeizuführen, hernach unter fortwährendem Rühren das Gemisch auf Raumtemperatur abkühlen zu lassen.

Es übersteigt nicht das Können des Fachmannes, durch einfache Versuche den geeigneten Temperaturbereich zu ermitteln, innerhalb dessen ein gegebenes Gemisch Phaseninversion durchlaufen kann. Überlicherweise ist dieser Temperaturbereich zwischen 70 und 95° C zu wählen, kann im Einzelfalle auch darüber oder darunter angesiedelt sein.

In der Praxis ist möglich und gegebenenfalls sogar vorteilhaft, bei der Herstellung einer erfindungsgemäßen Mikroemulsion den Temperaturbereich, der dem Phaseninversionsbereich zugeordnet werden kann, auch zu übersteigen, da beim Abkühlen auf Raumtemperatur dieser Bereich dann zwangsläufig durchlaufen wird.

Die Praxis der Herstellung einer erfindungsgemäßen Emulsion besteht vorteilhaft darin, nach Auswahl geeigneter Rohstoffe, d.h., Wasser- und Ölphase, ein oder mehrere Emulgatoren vom Typ A, letzterer oder letztere vorliegend in Konzentrationen, bei welchen Phaseninversion für das gegebene Gemisch möglich ist, und gegebenenfalls weitere Substanzen, die Einzelkomponenten unter Rühren auf eine Temperatur zu erwärmen, bei welcher Phaseninversion für das gegebene Gemisch möglich ist, und durch Erhöhung oder Erniedrigung des pH-Wertes des Gemisches Phaseninversion herbeizuführen, hernach unter fortwährendem Rühren das Gemisch auf Raumtemperatur abkühlen zu lassen. Ein oder mehrere zwischengeschaltete Homogenisierungsschritte sind vorteilhaft, aber nicht zwingend notwendig.

Eine weitere vorteilhafte Ausführungsform des erfindungsgemäßen Verfahrens besteht darin, nach der Auswahl geeigneter Rohstoffe, d.h., Wasser- und Ölphase, ein oder mehrere Emulgatoren vom Typ A letzterer oder letztere vorliegend in Konzentrationen, bei welchen Phaseninversion für das gegebene Gemisch möglich ist, und gegebenenfalls weitere Substanzen, die Einzelkomponenten unter Rühren auf einen pH-Wert zu bringen, bei welchem Phaseninversion für das gegebene Gemisch möglich ist, und durch Erhöhung der Temperatur des Gemisches Phaseninversion herbeizuführen, hernach unter fortwährendem Rühren das Gemisch auf Raumtemperatur abkühlen zu lassen. Ein oder

mehrere zwischengeschaltete Homogenisierungsschritte sind vorteilhaft, aber nicht zwingend notwendig.

Eine dritte vorteilhafte Ausführungsform des erfindungsgemäßen Verfahrens besteht dann, nach der Auswahl geeigneter Rohstoffe, d.h., Wasser- und Ölphase, ein oder mehrere Emulgatoren vom Typ A und gegebenenfalls weitere Substanzen, die Einzelkomponenten unter Rühren auf einen pH-Wert und eine Temperatur zu bringen, bei welchen Phaseninversion für das gegebene Gemisch möglich ist, und durch Zugabe des Emulgators A oder der Emulgatoren A zum Gemisch Phaseninversion herbeizuführen, hernach unter fortwährendem Rühren das Gemisch auf Raumtemperatur abkühlen zu lassen. Ein oder mehrere zwischengeschaltete Homogenisierungsschritte sind vorteilhaft, aber nicht zwingend notwendig.

In der Praxis ist möglich und gegebenenfalls sogar vorteilhaft, bei der Herstellung einer erfindungsgemäßen Emulsion den Temperaturbereich, der dem Phaseninversionsbereich zugeordnet werden kann auch zu übersteigen, da beim Abkühlen auf Raumtemperatur dieser Bereich dann zwangsläufig durchlaufen wird.

Es ist vorteilhaft, Zubereitungen gemäß der Erfindung zu puffern, bevorzugt im Bereich um pH = 4,5 - 5,5, insbesondere pH = 5.

Kosmetische und dermatologische Zubereitungen gemäß der Erfindung enthalten anorganische Pigmente, welche röntgenamorph oder nichtröntgenamorph sind, auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$, Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Röntgenamorphe Oxidpigmente sind Metalloxide oder Halbmetalloxide, welche bei Röntgenbeugungsexperimenten keine oder keine erkenntliche Kristallstruktur erkennen lassen. Oftmals sind solche Pigmente durch Flammenreaktion erhältlich, beispielsweise dadurch, daß ein Metall- oder Halbmetallhalogenid mit Wasserstoff und Luft (oder reinem Sauerstoff) in einer Flamme umgesetzt wird.

In kosmetischen, dermatologischen oder pharmazeutischen Formulierungen werden röntgenamorphe Oxidpigmente als Verdickungs- und Thixotropierungsmittel, Fließhilfsmittel, zur Emulsions- und Dispersionsstabilisierung und als Trägersubstanz (beispielsweise zur Volumenerhöhung von feinteiligen Pulvern oder Pudern) eingesetzt.

Bekannte und in der kosmetischen oder dermatologischen Galenik oftmals verwendete röntgenamorphe Oxidpigmente sind die Siliciumoxide des Typs Aerosil® (CAS-Nr. 763 1-86-9. Aerosile®, erhältlich von der Gesellschaft DEGUSSA, zeichnen sich durch geringe Partikelgröße (z.B. zwischen 5 und 40 nm) aus, wobei die Partikel als kugelförmige Teilchen sehr einheitlicher Abmessung anzusehen sind. Makroskopisch sind Aerosile® als lockere, weiße Pulver erkenntlich. Im Sinne der vorliegenden Erfindung sind röntgenamorphe Siliciumdioxidpigmente besonders vorteilhaft, und unter diesen gerade solche des Aerosil®-Typs bevorzugt.

Vorteilhafte Aerosil®-Typen sind beispielsweise Aerosil® OX50, Aerosil® 130, Aerosil® 150, Aerosil® 200, Aerosil® 300, Aerosil® 380, Aerosil® MOX 80, Aerosil® MOX 170, Aerosil® COK 84, Aerosil® R 202, Aerosil® R 805, Aerosil® R 812, Aerosil® R 972, Aerosil® R 974, Aerosil® R976.

Erfindungsgemäß vorteilhaft enthalten kosmetische oder dermatologische Lichtschutzzubereitungen 0,1 bis 20 Gew.-%, vorteilhaft 0,5 bis 10 Gew.-%, ganz besonders bevorzugt 1 bis 5 Gew.-% röntgenamorphe Oxidpigmente.

Die nichtröntgenamorphen anorganischen Pigmente liegen erfindungsgemäß vorteilhaft in hydrophober Form vor, d.h., daß sie oberflächlich wasserabweisend behandelt sind. Diese Oberflächenbehandlung kann dann bestehen, daß die Pigmente nach an sich bekannten Verfahren mit einer dünnen hydrophoben Schicht versehen werden.

Eines solcher Verfahren besteht beispielsweise dann, daß die hydrophobe Oberflächenschicht nach einer Reaktion gemäß

$$n\ TiO_2 + m\ (RO)_3Si\text{-}R' \text{ -> } n\ TiO_2\ \text{(oberfl.)}$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma Degussa erhältlich.

Die Gesamtmenge an anorganischen Pigmenten, insbesondere hydrophoben anorganischen Mikropigmenten in den fertigen kosmetischen oder dermatologischen Zubereitungen wird vorteilhaft aus dem Bereich von 0,1 - 30 Gew.-%, bevorzugt 0,1 - 10,0 Gew.-% gewählt, bezogen auf das Gesamtgewicht der Zubereitungen.

Die Emulgatoren A werden bevorzugt gewählt aus der Gruppe der Emulgatoren, welche gute Protonendonoren bzw. Protonenakzeptoren darstellen, wobei gewährleistet sein muß, daß ihre Lipophilie entweder abhängig ist vom pH-Wert, dergestalt daß durch Erhöhung oder Senkung des pH-Wertes die Lipophille zunimmt oder abnimmt, wobei unerheblich ist, welche der beiden Möglichkeiten der Änderung der Lipophilie durch die Erhöhung bzw. Senkung

des pH-Wertes bewirkt wird, oder ihre Lipophilie abhängig ist von der Temperatur, dergestalt, daß die Lipophilie mit steigender Temperatur zunimmt und deren Hydrophilie mit sinkender Temperatur zunimmt, oder ihre Lipophilie abhängig ist von pH-Wert und der Temperatur, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und daß die Lipophilie mit steigender Temperatur zunimmt und deren Hydrophilie mit sinkender Temperatur zunimmt.

Die erfindungsgemäßen Emulsionen sind vorteilhaft dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

Besonders vorteilhaft werden der oder die Emulgatoren A gewählt aus der Gruppe der mono-, oligo und polyethoxylierten Verbindungen, insbesondere der polyethoxylierten einoder mehrbasigen Alkohole oder Fettsäuren, beispielsweise

- der Fettalkoholethoxylate
- der ethoxylierten Wollwachsalkohole,
- der Polyethylenglycolether der allgemeinen Formel $R-O-(-CH_2-CH_2-O-)_n-R'$,
- der Fettsäureethoxylate der allgemeinen Formel $R-COO-(-CH_2-CH_2-O-)_n-H$,
- der veretherten Fettsäureethoxylate der allgemeinen Formel $R-COO-(-CH_2-CH_2-O-)_n-R'$,
- der veresterten Fettsäureethoxylate der allgemeinen Formel $R-COO-(-CH_2-CH_2-O-)_n-C(O)-R'$,
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel $R-O-(-CH_2-CH_2-O-)_n-CH_2-COOH$ nd n eine Zahl von 5 bis 30 darstellen,
- der Polyoxyethylensorbitolfettsäureester,
- der Alkylethersulfate der allgemeinen Formel $R-O-(-CH_2-CH_2-O-)_n-SO_3-H$
- der Fettalkoholpropoxylate der allgemeinen Formel $R-O-(-CH_2-CH(CH_3)-O-)_n-H$,
- der Polypropylenglycolether der allgemeinen Formel $R-O-(-CH_2-CH(CH_3)-O-)_n-R'$,
- der propoxylierten Wollwachsalkohole,
- der veretherten Fettsäurepropoxylate $R-COO-(-CH_2-CH(CH_3)-O-)_n-R'$,
- der veresterten Fettsäurepropoxylate der allgemeinen Formel $R-COO-(-CH_2-CH(CH_3)-O-)_n-C(O)-R'$,
- der Fettsäurepropoxylate der allgemeinen Formel $R-COO-(-CH_2-CH(CH_3)-O-)_n-H$,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel $R-O-(-CH_2-CH(CH_3)O-)_n-CH_2-COOH$
- der Alkylethersulfate bzw. die diesen Sulfaten zugrundeliegenden Säuren der allgemeinen Formel $R-O-(CH_2-CH(CH_3)-O-)_n-SO_3-H$
- der Fettalkoholethoxylate/propoxylate der allgemeinen Formel $R-O-X_n-Y_m-H$,
- der Polypropylenglycolether der allgemeinen Formel $R-O-X_n-Y_m-R'$,
- der veretherten Fettsäurepropoxylate der allgemeinen Formel $R-COO-X_n-Y_m-R'$,
- der Fettsäureethoxylate/propoxylate der allgemeinen Formel $R-COO-X_n-Y_m-H$,.

Erfindungsgemäß besonders vorteilhaft werden die eingesetzten Emulgatoren A gewählt aus der Gruppe der Substanzen mit HLB-Werten von 11 - 18, ganz besonders vorteilhaft mit HLB-Werten von 14,5 - 15,5, sofern die Emulgatoren A gesättigte Reste R und R' aufweisen. Weisen die Emulgatoren A ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetylstearylalkohole) zu wählen. Insbesondere bevorzugt sind:

Polyethylenglycol(13)stearylether (Steareth-13), Polyethylenglycol(14)stearylether (Steareth- 14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol (19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenlglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glycerylaurat, Polyethylenglycol(21)glycerylaurat, Polyethylenglycol(22)glycerylaurat, Polyethylenglycol(23)glycerylaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Vorteilhaft werden die Coemulgatoren gewählt aus der Gruppe der Sorbitanester und Saccharoseester, insbesondere der verzweigten und unverzweigten Alkylester und Alkenylester mit Kohlenstoffketten von 4 - 24 Kohlenstoffatomen, bevorzugt Sorbitanstearat, Sorbitanoleat, Glycerylsorbitanstearat, Saccharosemonostearat, Saccharosemonolaurat, Saccharosepalmitat.

Vorteilhaft können die Coemulgatoren gewählt aus der Gruppe der Monoglycennmonocarbonsäure-monoester gewählt werden, insbesondere solche, welche durch die Strukturen

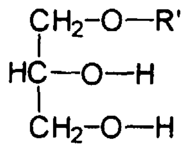

bzw.

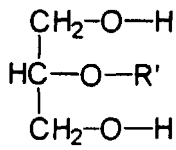

gekennzeichnet sind, wobei R' einen verzweigten oder unverzweigten Acylrest mit 6 - 14 Kohlenstoffatomen darstellt. Vorteilhaft wird R' gewählt aus der Gruppe der unverzweigten Acylreste.

Die diesen Estern zugrundeliegenden Säuren sind die

| | | |
|---|---|---|
| Hexansäure | (Capronsäure) | (R' = $-C_5H_{11}$), |
| Heptansäure | (Önanthsäure) | (R' = $-C_6H_{13}$), |
| Octansäure | (Caprylsäure) | (R' = $-C_7H_{15}$), |
| Nonansäure | (Pelargonsäure) | (R' = $-C_8H_{17}$), |
| Decansäure | (Caprinsäure) | (R' = $-C_9H_{19}$), |
| Undecansäure | | (R' = $-C_{10}H_{21}$), |
| 10-Undecensäure | (Undecylensäure) | (R' = $-C_{10}H_{19}$), |
| Dodecansäure | (Laurinsäure) | (R' = $-C_{11}H_{23}$), |
| Tridecansäure | | (R' = $C_{12}H_{25}$), |
| Tetradecansäure | (Myristinsäure) | (R' = $-C_{13}H_{27}$), |

Besonders vorteilhaft stellt R' den Octanoylrest (Caprylsäurerest) bzw. den Decanoylrest (Caprinsäurerest) dar, wird also also durch die Formeln

$R' = -C_7H_{15}$ bzw. $R' = -C_9H_{19}$

repräsentiert.

Vorteilhaft können die Emulgatoren des Typs A auch aus der Gruppe der Di- und Triglycerin-monocarbonsäure-monoester gewählt werden. Erfindungsgemäß liegen die Di- bzw. Triglycerineinheiten der erfindungsgemäßen Diglycerin-monocarbonsäure-monoester bzw. Tnglycerin-monocarbonsäure-monoester als lineare, unverzweigte Moleküle, also über die jeweiligen OH-Gruppen in 1- bzw. 3-Stellung veretherte "Monoglycerinmoleküle" vor.

Ein geringer Anteil zyklischer Di- bzw. Triglycerineinheiten sowie über die OH-Gruppen in 2-Stellung veretherte Glycerinmoleküle kann geduldet werden. Es ist jedoch von Vorteil, solche Verunreinigungen so gering wie nur möglich zu halten.

Die erfindungsgemäßen Monocarbonsäuremonoester sind bevorzugt durch folgende Struktur gekennzeichnet:

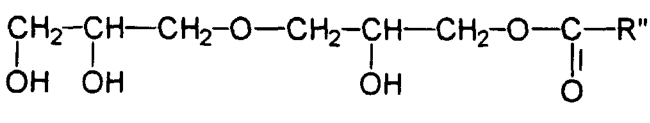

wobei R“ einen Kohlenwasserstoffrest, vorteilhaft einen verzweigten oder unverzweigten Alkyl- oder Alkenylrest von 5 bis 17 C-Atomen darstellt.

Die erfindungsgemäßen Monocarbonsäureester des Triglycerins sind bevorzugt durch folgende Struktur gekennzeichnet:

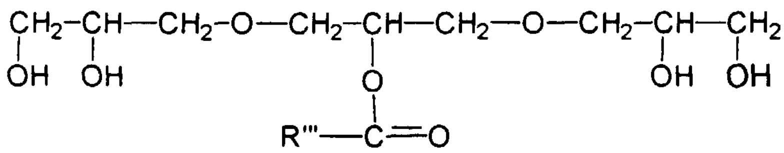

wobei R'" einen Kohlenwasserstoffrest, vorteilhaft einen verzweigten oder unverzweigten Alkyl- oder Alkenylrest Alkylrest von 5 bis 17 C-Atomen darstellt.

Die diesen Estern zugrundeliegenden Säuren sind die

| | | |
|---|---|---|
| Hexansäure | (Capronsäure) | (R“ bzw. R'“ = $-C_5H_{11}$), |
| Heptansäure | (Önanthsäure) | (R“ bzw. R'“= $-C_6H_{13}$), |
| Octansäure | (Caprylsäure) | (R“ bzw. R'“ =$-C_7H_{15}$), |
| Nonansäure | (Pelargonsäure) | (R“ bzw. R'“= $-C_8H_{17}$), |
| Decansäure | (Caprinsäure) | (R“ bzw. R'“ = $-C_9H_{19}$), |
| Undecansäure | | (R“ bzw. R'“ = $-C_{10}H_{21}$), |
| 10-Undecensäure | (Undecylensäure) | (R“ bzw. R'“ = $-C_{10}H_{19}$), |
| Dodecansäure | (Laurinsäure) | (R“ bzw. R'“ = $-C_{11}H_{23}$), |
| Tridecansäure | | (R“ bzw. R'“ = $-C_{12}H_{25}$), |
| Tetradecansäure | (Myristinsäure) | (R“ bzw. R'“ = $-C_{13}H_{27}$), |
| Pentadecansäure | | (R“ bzw. R'“ = $-C_{14}H_{29}$), |
| Hexadecansäure | (Palmitinsäure) | (R“ bzw. R'“ = $-C_{15}H_{31}$), |
| Heptadecansäure | (Margarinsäure) | (R“ bzw. R'“ = $-C_{16}H_{33}$), |
| Octadecansäure | (Stearinsäure) | (R“ bzw. R'“ = $-C_{17}H_{35}$). |

Besonders günstig werden R“ und R'" gewählt aus der Gruppe der unverzweigten Alkylreste mit ungeraden C-Zahlen, insbesondere mit 9, 11 und 13 C-Atomen.

Im allgemeinen sind die Monocarbonsäure-monoester des Diglycerins denen des Triglycerins bevorzugt.

Erfindungsgemäß ganz besonders günstig sind

| | | |
|---|---|---|
| Diglycerinmonocaprinat | (DMC) | R“ = 9 |
| Triglycerinmonolaurat | (TML) | R'“ = 11 |
| Diglycerinmonolaurat | (DML) | R“ = 11 |
| Triglycerinmonomyristat | (TMM) | R'“ = 13 |

Als bevorzugter erfindungsgemäßer Monocarbonsäuremonoester des Diglycerins hat sich das Diglycerinmonocaprinat (DMC) erwiesen.

Nach einer vorteilhaften Ausführungsform der vorliegenden Erfindung wird ein zusätzlicher Anteil an in anderen Stellen verestertem Di- oder Triglycerin, ebenso wie gegebenenfalls ein Anteil an den verschiedenen Diestern

des Di- bzw. Triglycerins verwendet.

Vorteilhaft sind auch Triglyceryldiisostearat (Nomenklatur analog CTFA: Polyglyceryl-3-di-isostearat), Isostearyldiglycerylsuccinat, Diglycerylsesquiisostearat (Nomenklatur analog CTFA: Polyglyceryl-2-sesquiisostearat), Triglycerylpolyhydroxystearat (Nomenklatur analog CTFA: Polyglyceryl-2-polyhydroxystearat).

Auch Cetylstearylisononanoat, Dicocoyl-pentaerythrityl-distearylcitrat, ferner die Methiconcopolyole, Cyclomethiconcopolyle, Alkylmethiconcopolyole, insbesondere Laurylmethiconcopolyol, Cetyldimethiconcopolyol, haben sich erfindungsgemäß als vorteilhaft erwiesen.

Besonders vorteilhaft werden der oder die Coemulgatoren gewählt aus der Gruppe der verzweigten oder unverzweigten Alkylmonocarbonsäuren, Alkenylmonocarbonsäuren und Alkylendicarbonsäuren mit 4 bis 30 Kohlenstoffatomen, insbesondere Stearinsäure, Ölsäure, Bernsteinsäure, Hexansäure (Capronsäure), Heptansäure (Önanthsäure), Octansäure (Caprylsäure), Nonansäure (Pelargonsäure), Decansäure (Caprinsäure), Undecansäure, Undecensäure (Undecylensäure), Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Isostearinsäure, Behensäure. Es ist auch vorteilhaft, die Emulgatoren A aus der Gruppe der kosmetisch bzw. pharmazeutisch akzeptablen Salze der vorstehend genannten Carbonsäuren zu wählen, insbesondere der Alkali-, Ammonium-, Monoalkylammonium, Dialkylammonium-, Trialkylammonium- und Tetraalkylammoniumsalze.

Erfindungsgemäß vorteilhaft können als Coemulgatoren Siliconemulgatoren, und zwar diese vorteilhaft aus der Gruppe grenzflächenaktive Substanzen aus der Gruppe der Alkylmethiconcopolyole und/oder Alkyl-Dimethiconcopolyole gewählt werden, bevorzugt aus der Gruppe der Verbindungen, welche gekennzeichnet sind durch die folgende chemische Struktur:

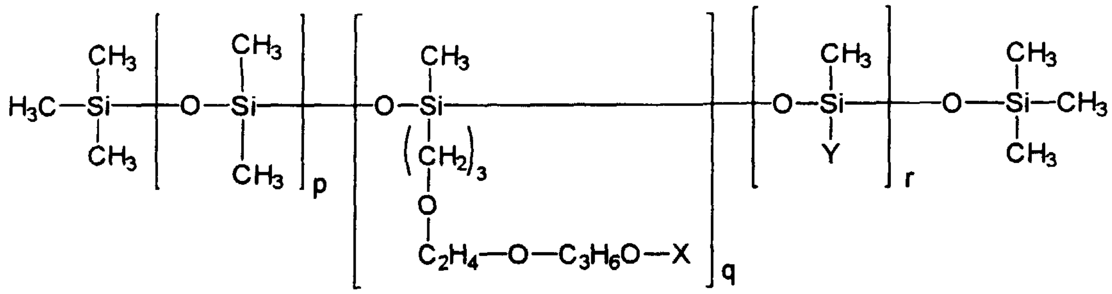

bei welcher X und Y unabhängig voneinander gewählt werden aus der Gruppe H sowie der verzweigten und unverzweigten Alkylgruppen, Acylgruppen und Alkoxygruppen mit 1 - 24 Kohlenstoffatomen, p eine Zahl von 0 - 200 darstellt, q eine Zahl von 1 - 40 darstellt, und r eine Zahl von 1 - 100 darstellt.

Ein Beispiel für besonders vorteilhaft im Sinne der vorliegenden Erfindung zu verwendende Silikonemulgatoren sind Dimethiconcopolyole, welche von der Gesellschaft Th. Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an erfindungsgemäß verwendeten Siliconemulgatoren in den erfindungsgemäßen kosmetischen oder dermatologischen Zubereitungen wird vorteilhaft aus dem Bereich von 0,1 - 10,0 Gew.-%, bevorzugt 0,5 - 5,0 Gew.-% gewählt, bezogen auf das Gesamtgewicht der Zubereitungen.

Es ist erfindungsgemäß möglich, die Einsatzmengen von an sich in Ölkomponenten schwerlöslichen oder unlöslichen UV-Flltern, insbesondere 4,4',4"-(1,3, 5-Triazin-2,4,6-triyltriimino)-tris-benzoesäure-tris(2-ethylhexylester), aber auch 2-Phenylbenzimidazol-5-sulfonsäure bzw. deren Salze in kosmetischen oder dermatologischen Zubereitungen gegenüber dem Stande der Technik zu vervielfachen.

Die Gesamtmenge an in Ölkomponenten an sich schwerlöslichen UV-Filtersubstanzen, insbesondere 4,4',4"-(1,3,5-Triazin-2,4,6-triyltriimino)-tris-benzoesäure-tris(2-ethylhexylester), aber auch 2-Phenylbenzimidazol-5-sulfonsäure bzw. deren Salze in den fertigen kosmetischen oder dermatologischen, Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Es ist erfindungsgemäß vorteilhaft, in den erfindungsgemäßen Zubereitungen zusätzliche öllösliche UVA-Filter und/oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen.

Die zusätzlichen UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 4,4',4''-(1,3,5-Triazin-2,4,6-triyltriimino)-tris-benzoesäure-tris(2-ethylhexylester).

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst,
- Sulfonsäure-Derivate von Benzophenonen, vorzugsweise 2-Hydroxy-4-methoxybenzophenon-5-sulfonsäure und deren Salze,
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, zusätzliche UVA-Filter in den erfindungsgemäßen Zubereitungen einzusetzen, welche bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan- 1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen

Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, $ZnSO_4$) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexylaurat, n-Decyloleat, Isooctylstearat, Isononylstearat, Isononylisononanoat, 2-Ethylhexylpalmitat, 2-Ethylhexylaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Nachfolgend soll kurz noch auf einige Besonderheiten und Unterschiede der Voraussetzungen für erfindungsgemäß O/W-Emulsionen und O/W-Mikroemulsionen eingegangen werden.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Erfindungsgemäß wird für O/W-Mikroemulsionen die Ölphase vorteilhaft gewählt aus der Gruppe der polaren Ölkomponenten, welche eine Polarität zwischen 10 und 30 mN/m aufweisen, wobei gewährleistet sein muß, daß mindestens eine nichtpolare Ölkomponente vorhanden ist.

Vorteilhafte O/W-Mikroemulsionen werden erhalten, wenn die Ölphase gewählt wird aus der Gruppe der polaren Ölkomponenten, besonders bevorzugt aus der Gruppe der natürlichen, synthetischen oder halbsynthetischen Ölkomponenten, welche eine Polarität zwischen 10 und 20 mN/m aufweisen, wobei gewährleistet sein muß, daß mindestens eine nichtpolare Ölkomponente vorhanden ist.

Vorteilhaft ist auch, polare pflanzliche Öle als polare Öle der erfindungsgemäßen O/W-Emulsionen zu verwenden. Die pflanzlichen Öle können vorteilhaft gewählt werden aus der Gruppe der Öle der Pflanzenfamilien Euphorbiaceae, Poaceae, Fabaceae, Brassicaceae, Pedalaceae, Asteraceae, Linaceae, Flacourticaceae, Violales, vorzugsweise gewählt aus der Gruppe natives Rizinusöl, Weizenkeimöl, Traubenkernöl, Kukuinußöl, Safloröl, Distelöl, Nachtkerzenöl und weiteren Ölen, welche mindestens 1,5 Gew.-% an Linolsäureglyceriden enthalten.

Der Zusatz von Elektrolyten bewirkt eine Veränderung des Löslichkeitsverhaltens eines hydrophilen Emulgators. Die hydrophilen Emulgatoren mit den vorab beschriebenen Strukturen bzw. Eigenschaften durchlaufen eine

partielle Phaseninversion, in der es zu einer Solubilisierung von Wasser durch die Ölphase kommt, welche in einer stabilen Mikroemulsion resultiert.

Die erfindungsgemäßen Mikroemulsionen enthalten daher vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des Elektrolyten oder der Elektrolyte sollte etwa 0,01 - 10,0 Gew.-%, besonders vorteilhaft etwa 0,03 - 8,0 Gew.% betragen, bezogen auf das Gesamtgewicht der Zubereitung.

Die Emulgatoren des Typs A können landsläufig als O/W-Emulgatoren angesehen werden. Ein Gehalt von etwa 5 - 10 Gew.-% an üblichen W/O-Emulgatoren fördert in vorteilhafter Weise die Bildung von O/W/O-Emulsionen, ein Gehalt von deutlich über 10 Gew.-% an solchen Emulgatoren führt zur Destabilisierung der O/W/O-Emulsionen.

Ferner ist gewünschtenfalls für die Herstellung erfindungsgemäßer O/W/O-Emulsionen von Vorteil, hydrophile und/oder lipophile Gelbildner einzusetzen. Diese tragen zwar in der Regel nicht zur Bildung multipler Tröpfchen bei, förden aber die Stabilität einmal gebildeter multipler Tröpfchen.

Wenn bei einem erfindungsgemäßen Herstellungsverfahren für O/W/O-Emulsionen der pH-Wert variiert werden soll, um ein ansonsten vorbestimmtes System in den Phaseninversionsbereich zu bringen, so ist von Vorteil, zu Beginn des Verfahrens zunächst möglichst geringe Elektrolytkonzentration in der Wasserphase einzusetzen, möglichst zunächst ganz darauf zu verzichten. Weiterhin ist von Vorteil, den Emulgator A in der Ölphase vorzulegen, beispielsweise für Stearinsäure im Konzentrationsbereich von 0,5 - 5 Gew.-%, insbesondere 2 Gew.-%. Vorteilhaft ist die Gegenwart eines nicht unter die Definition des Emulgators A fallenden Emulgators im Konzentrationsbereich von ca. 5 - 10 Gew.-%, insbesondere etwa 7 Gew.-%.

Die Variation des pH-Wertes sollte vorteilhaft erst erfolgen, wenn die W/O-Emulsion sich gebildet hat, beispielsweise durch Zufügen von NaOH.

Dabei liegt im allgemeinen Fachwissen des Fachmannes und bedarf keinerlei erfinderischen Dazutuns, für einen gegebenen Emulgator oder ein gegebenes Emulgatorsystem in einem gegebenen Wasser/Ölphasensystem den Temperatur- bzw. pH-Bereich zu ermitteln, in welchem Phaseninversion stattfindet. Als allgemeiner Richtwert für den PIT bei üblichen Emulgatorkonzentrationen kann ein Temperaturbereich von etwa 40 - 90° C angegeben werden. Im allgemeinen sinkt der PIT bei steigender Emulgatorkonzentration.

Es können während dieses Verfahrens gewünschtenfalls ferner die in der Kosmetik oder medizinischen Galenik üblichen Grund-, Hufs-, Zusatz-, und/oder Wirkstoffe zugegeben werden. Es ist dem Fachmann klar, zu welchem Zeitpunkt solche Stoffe dem Prozeß beigegeben werden können, ohne daß die Eigenschaften der zu erzielenden Emulsion wesentlich beeinträchtigt werden.

Die nachfolgenden Beispiele sollen das Wesen der vorliegenden Erfindung näher umreißen, ohne die Erfindung einzuschränken.

| Beispiel 1: Selbstbräunungsspray mit intensiver Bräunungsleistung | |
|---|---|
| Cetylstearylisononanoat | 8,00 |
| Ceteareth-20 | 6,00 |
| Dihydroxyaceton | 5,00 |
| Glycerin | 5,00 |
| Dicaprylylether | 4,00 |
| Vitamin E Acetat | 0,50 |
| Glycerylstearat | 2,00 |

(fortgesetzt)

| Beispiel 1: Selbstbräunungsspray mit intensiver Bräunungsleistung | |
|---|---|
| Natriumcitrat | 0,50 |
| Citronensäure | 0,20 |
| Farbstoffe, Parfum, Konservierungsmittel | 0,50 |
| Parfüm | q.s. |
| Wasser | Ad. 100,00 |

| Beispiel 2: Selbstbräunungsspray mit intensiver Bräunungsleistung | |
|---|---|
| Cetylstearylisononanoat | 4.0000 |
| Ceteareth-15 | 6.0000 |
| Dihydroxyaceton | 5.0000 |
| Glycerin | 5.0000 |
| Dicaprylylether | 5.0000 |
| Vitamin E Acetat | 0.5000 |
| Stearinsäure | 2.3000 |
| Natriumhydroxid | 0.1070 |
| Citronensäure | 0.2000 |
| Farbstoffe, Parfum, Konservierungsmittel | q.s. |
| Wasser | Ad. 100 |

| Beispiel 3: Selbstbräunungsspray mit mittlerer Bräunungsleistung | |
|---|---|
| C12-15 Alkylbenzoate | 7.5000 |
| Glycerin | 5.0000 |
| Cetylstearylisononanoat | 5.0000 |
| Dihydroxyaceton | 2.0000 |
| Sorbitanmonoisostearat | 4.0000 |
| Butylenglycoldicaprylat/dicaprat | 2.5000 |
| Vitamin E -Acetat | 0.5000 |
| Cetylstearyl Alcohol | 1.5000 |
| Farbstoffe, Parfum, Konservierungsmittel | q.s. |
| Wasser | Ad. 100 |

| Beispiel 4: Selbstbräunungsspray mit schwacher Bräunungsleistung | |
|---|---|
| Dihydroxyaceton | 1.0000 |
| Glycerin | 5.0000 |
| Dicaprylylether | 5.0000 |
| Isoceteth-20 | 4.8000 |
| Cetylstearylalkohol | 2.4000 |
| DMDM Hydantoin | 0.4000 |
| Farbstoffe, Parfum, Konservierungsmittel | q.s. |
| Wasser | Ad. 100 |

| Beispiel 5: Selbstbräunungsspray mit zusätzlichem Antioxidanzschutz | |
|---|---|
| Dihydroxyaceton | 5.0000 |
| $C_{12-15}$ Alkylbenzoate | 4.5000 |
| Ceteareth-12 | 4.0000 |
| Cetylstearylisononanoat | 2.5000 |
| Glycerin | 2.0000 |
| Dimethicon | 1.5000 |
| Dicaprylylether | 1.0000 |
| Glycerylisostearat | 2.0000 |
| Cetylalcohol | 1.0000 |
| DMDM Hydantoin | 0.2000 |
| Glucosylrutin | 0.5000 |
| Parfüm | q.s. |
| Wasser | Ad. 100 |

| Beispiel 6: Selbstbräunungsspray mit hohem UV-Schutz | |
|---|---|
| Dicaprylylether | 3.0000 |
| Glycerin | 5.0000 |
| Octyltriazon | 1.0000 |
| Dioctylbutamidotriazon | 2.0000 |

(fortgesetzt)

| Beispiel 6: Selbstbräunungsspray mit hohem UV-Schutz | |
|---|---|
| Anisotriazin | 1.0000 |
| Bisimidazylat | 0.5000 |
| Titandioxid | 0.5000 |
| Dihydroxyaceton | 5.0000 |
| Ceteareth-20 | 4.0000 |
| Butylenglycoldicaprylat/dicaprat | 5.0000 |
| Vitamin E -Acetat | 0.5000 |
| Cetylstearylalcohol | 1.5000 |
| Farbstoffe, Parfum, Konservierungsmittel | q.s. |
| Parfüm | q.s. |
| Wasser | Ad. 100 |

| Beispiel 7: Selbstbräunungsspray mit niedrigem UV-Schutz | |
|---|---|
| Glycerin | 5.0000 |
| Anisotriazin | 1.0000 |
| Butylmethoxydibenzoylmethan | 0.5000 |
| Bisimidazylate | 0.5000 |
| Dihydroxyaceton | 5.0000 |
| Ceteareth-20 | 4.0000 |
| Butylenglycoldicaprylat/dicaprat | 5.0000 |
| Vitamin E -Acetat | 0.5000 |
| Cetylstearylalcohol | 1.5000 |
| Farbstoffe, Parfum, Konservierungsmittel | q.s. |
| Wasser | Ad. 100 |

## Patentansprüche

**1.** Öl-in-Wasser-Emulsionen, insbesondere O/W-Mikroemulsionen

(a) enthaltend mindestens einen Emulgator (Emulgator A), gewählt aus der Gruppe der Emulgatoren mit folgenden Eigenschaften

- ihre Lipophilie ist entweder abhängig vom pH-Wert, dergestalt daß durch Erhöhung oder Senkung des pH-Wertes die Lipophilie zunimmt oder abnimmt, wobei unerheblich ist, welche der beiden Möglichkeiten der Änderung der Lipophilie durch die Erhöhung bzw. Senkung des pH-Wertes bewirkt wird, und/oder
- ihre Lipophilie ist abhängig von der Temperatur, dergestalt, daß die Lipophilie mit steigender Temperatur zunimmt und deren Hydrophilie mit sinkender Temperatur zunimmt,

(b) ferner gegebenenfalls weitere in der Ölphase oder der Wasserphase lösliche lösliche oder dispergierbare

Substanzen, darunter bevorzugt solche, gewählt aus der Gruppe der nicht unter die Definition des Emulgators A fallende Emulgatoren, insbesondere solche, die vorwiegend als W/O-Emulgatoren wirken,

(c) eine wirksame Menge an Dihydroxyaceton.

**2.** O/W-Makroemulsionen oder O/W-Mikroemulsionen nach Anspruch 1, dadurch gekennzeichnet, daß der Emulgator A oder die Emulgatoren A in Konzentrationen von 0,01 - 20 Gew.-%, bevorzugt 0,05 - 10 Gew.-%, besonders bevorzugt 0,1 - 5 Gew.-%, jeweils bezogen auf das Gesamtgewicht der Zusammensetzung, vorliegt oder vorliegen.

**3.** O/W-Makroemulsionen oder O/W-Mikroemulsionen nach Anspruch 1, dadurch gekennzeichnet, daß die Gesamtmenge an Dihydroxyaceton in den fertigen kosmetischen oder dermatologischen Zubereitungen aus dem Bereich von 0,1 - 10,0 Gew.-%, bevorzugt 0,5 - 6,0 Gew.-% gewählt wird, bezogen auf das Gesamtgewicht der Zubereitungen.

Fig. 1

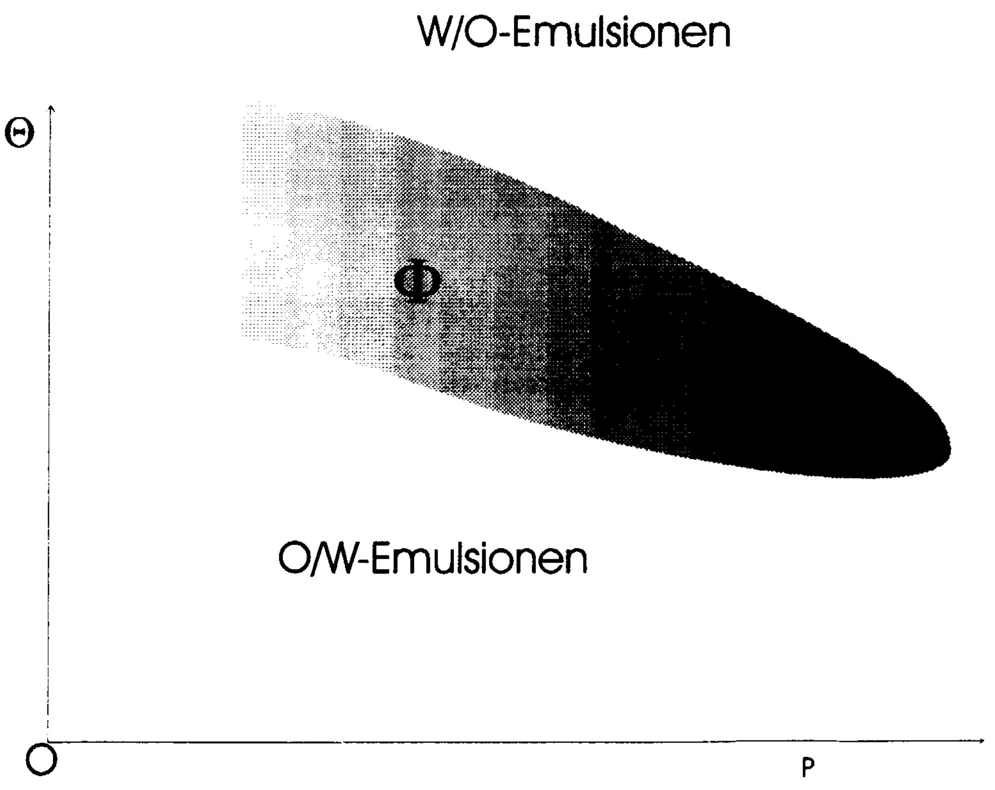

Fig. 2

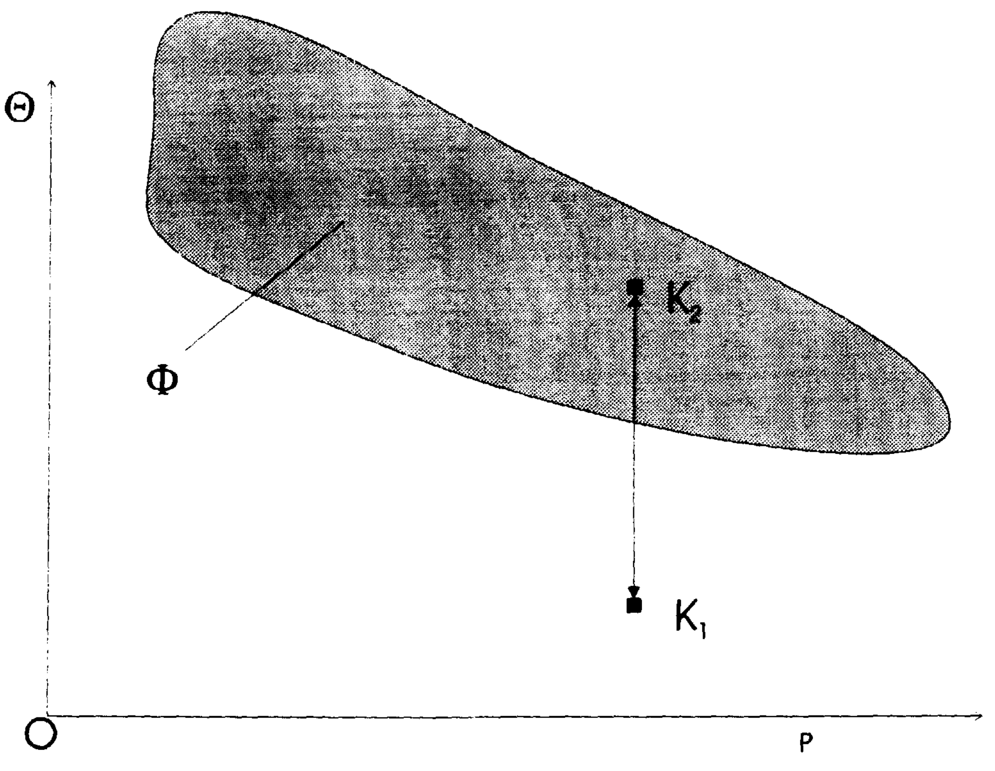

Fig. 3

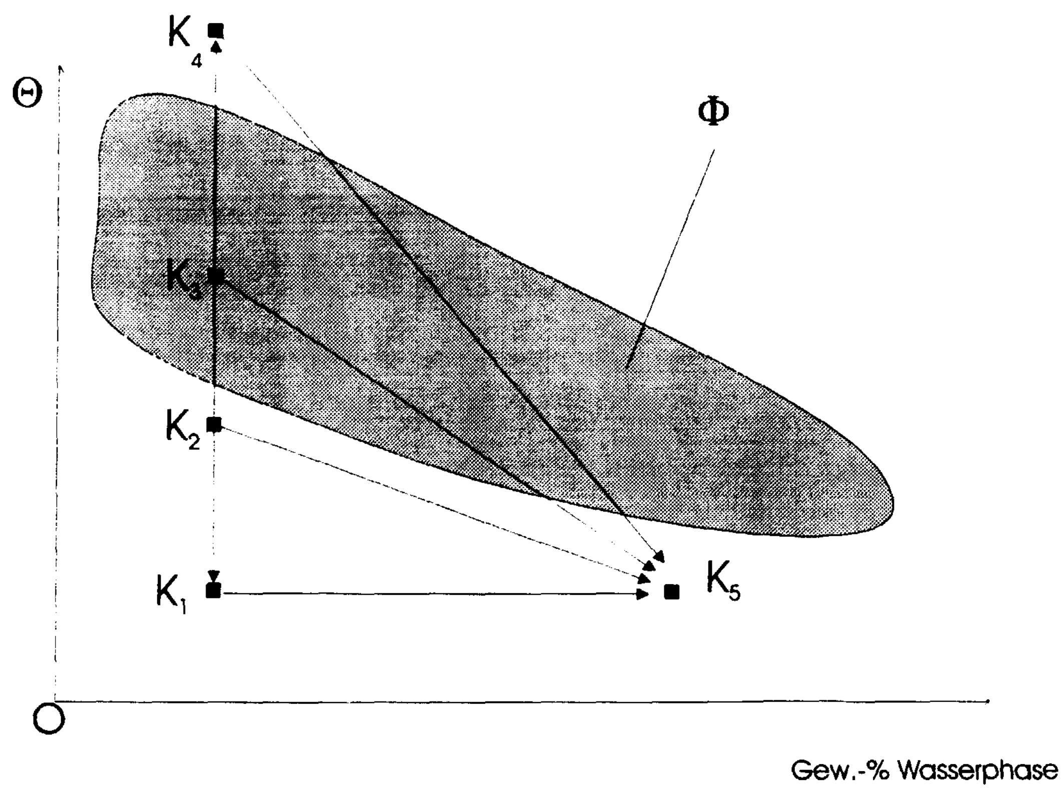

Fig. 4